# EUROPEAN PATENT SPECIFICATION

(11) **EP 1 896 038 B1**
(45) Date of publication and mention of the grant of the patent: **09.11.2016**
(21) Application number: 06741180.1
(22) Date of filing: 02.06.2006
(51) Int. Cl.: A61P 15/00, A61K 31/568, A61K 31/57, A61K 8/63, A61K 9/08, A61Q 15/00

(54) **METHOD AND COMPOSITION FOR TESTOSTERONE TRANSDERMAL DELIVERY**
VERFAHREN UND ZUSAMMENSETZUNG FÜR DIE TRANSDERMALE ABGABE VON TESTOSTERON
METHODE ET COMPOSITION POUR L'ADMINISTRATION TRANSDERMIQUE DE LA TESTOSTERONE

(30) Priority: 03.06.2005 AU 2005902902; 23.12.2005 US 752884 P
(43) Date of publication of application: 12.03.2008
(73) Proprietor: Acrux DDS Pty Ltd, West Melbourne, Victoria 3003 (AU)
(72) Inventor: DI PIETRO, Tony, Templestowe Lower, Victoria 3107 (AU); HUMBERSTONE, Andrew, Elsternwick, Victoria 3185 (AU); GONDA, Igor, South Yarra, Victoria 3141 (AU); WATKINSON, Adam, Williamtown, Victoria 3016 (AU); ROBINSON, Monique, View Bank, Victoria 3084 (AU); SETIAWAN, Kerrie, Wynn Vale, South Australia 5127 (AU); STONE, Carolyn, South Melbourne, Victoria 3205 (AU); WILKINS, Nina, Kensington, Victoria 3031 (AU)
(74) Representative: Schnappauf, Georg
(86) International application number: PCT/AU2006/000763
(87) International publication number: WO 2006/128255

(56) References cited:
- WO-A1-2004/091631
- WO-A1-2005/039531
- WO-A1-2005/049026
- WO-A2-2004/080413
- GB-A- 1 192 003
- US-A- 5 962 505
- US-A1- 2001 051 157
- US-A1- 2004 028 725
- US-A1- 2004 175 416
- US-A1- 2005 020 552
- US-A1- 2005 025 833
- US-A1- 2005 042 268
- US-A1- 2005 049 233
- BEN-GALIM E ET AL: "TOPICALLY APPLIED TESTOSTERONE AND PHALLIC GROWTH ITS EFFECT IN MALE CHILDREN WITH HYPOPITUITARISM AND MICROPHALLUS", AMERICAN JOURNAL OF DISEASES OF CHILDREN, AMERICAN MEDICAL SOCIETY, US, vol. 134, no. 3, 1 March 1980 (1980-03-01) , pages 296-298, XP001084709, ISSN: 0002-922X

## Description

### Field of the invention

The present invention relates to transdermal drug delivery compositions for use in the treatment of testosterone deficiency.

### Background of the invention

Efficient transdermal delivery of a physiologically active agent offers several clinical and patient advantages in treatment of disease.

Drug delivery by injection is traditionally the quickest route of administration to the systemic circulation, however the duration of action of is often short lived and the mode of delivery is invasive and painful. Transdermal drug delivery is receiving increased attention due to the ability of the administration regime to provide a controlled route for the release of an active agent to the systemic circulation.

However, transdermal drug delivery is complicated by the fact that the skin behaves as a natural barrier. As a result, the success of transdermal delivery system often relies on the ability of a composition to be able to penetrate the stratum corneum of the skin and thereby transport an active agent across the skin.

Problems with transdermal delivery arise particularly in cases where a drug is slow to be absorbed through the skin or where a relative high dose of drug needs to be absorbed. In these circumstances it is often necessary to apply a transdermal composition to a large area of skin in order to achieve the required dose.

One example of this problem is encountered in the administration of androgens such as testosterone. Recent estimates show that approximately 13 million men in the United States experience testosterone deficiency and less than 10% receive treatment for the condition. The daily dose required to maintain testosterone levels within the normal range is relatively high (typically 5-10 mg/day), thus it provides a challenge for transdermal delivery. Similar problems occur with progesterone. The previously used topical administration of testosterone, for example in treatment of hypogonadism, requires application of gel or cream to a wide area of the body and that the area of application remains covered. Application of composition over a wide area severely increases the risk of transfer from patient to partner or child, thus persons such as family members may be subjected to accidental dosing of the drug. This can have serious consequences.

Transdermal administration of some drugs may also be accompanied by undesirable side effects. For example, testosterone is responsible for increasing perspiration and producing perspiration related odour in the presence of the enzyme 5-alpha-reductase which converts testosterone to dihydrotestosterone (DHT). Scrotal skin has a relatively high level of 5-alpha-reductase and continuous trans-scrotal delivery of testosterone produces levels of DHT and DHT/testosterone ratios 4 to 5 fold greater than normal. Such abnormal levels and ratios may give rise to undesirable side effects.

Problems also arise with the location of application, and side effects associated with the location. Ahmed, S. R., et al. (J. Clin. Endocrinol. Metab. (1988) 66:546-557) and Findlay, J.C. (J. Clin. Endocrinol. Metab. (1989) 68:369-373) report that the 60 cm² ALZA trans-scrotal system delivers about 3.7 mg/day and produces low-normal testosterone levels in hypogonadal men. Such dosages are believed to be somewhat less than the amount needed to mimic endogenous production (5-10 mg/day).

Trans-scrotal delivery of testosterone is also associated with high dihydrotestosterone (DHT) and DHT/testosterone ratio levels and does not provide a level of testosterone delivery that mimics endogenous production. Further, scrotal skin is sensitive and as noted above, limited in area, which may result in discomfort and poor patient acceptance of this modality of delivery.

Many transdermal compositions utilise penetration enhancers to assist delivery of the pharmaceutical active across the skin. Increased cutaneous blood flow and subsequent heat production is also reported to assist. US2004028725 describes transdermal drug delivery compositions comprising testosterone, a volatile solvent, a penetration enhancer selected from octyl dimethyl-para-aminobenzoate or octyl salicylate and a viscosity modulating agent for use in the treatment of testosterone deficiency by non-occlusive application to an unspecified area of the skin.

US Patent 6,743,448 describes a topical testosterone formulation comprising arginine which is said to facilitate the production of nitrous oxide and enhance vasodilation.

However locally induced vasodilation and subsequent heating, particularly in areas having increased cutaneous blood flow such as the axilla is likely to cause excess perspiration and discomfort, due to the increased number of sweat glands present in such areas. This can be problematic for the subject on the basis that transdermal delivery of some pharmaceutical actives may be hindered by the use of antiperspirants and/or deodorants. This is undesirable because delivery may be compromised because of perspiration, resulting in inconsistent delivery and/or patient acceptability and compliance is hindered by instructions not to use antiperspirant or deodorant with drug delivery that promotes perspiration.

There is therefore a need for a method and composition for transdermal administration which provides rapid, consistent delivery and allows the area of application to be minimised, as well as being convenient for subjects to use desirably with reduced side effects to subjects and others with whom they come in contact.

### Summary of the invention

In a first aspect, the present invention provides the use of a transdermal drug delivery composition comprising (a) testosterone; (b) at least one volatile solvent; (c) a penetration enhancer which is one or a mixture of octyl dimethyl-para-aminobenzoate, octyl para-methoxycinnamate and octyl salicylate; and (d) at least one viscosity modulating agent, in manufacture of a pharmaceutical composition for the treatment of testosterone deficiency in a subject by application to at least one axilla to deliver testosterone systemically; and wherein the transdermal drug delivery composition is for application without being covered by adhesives, adhered patches, adhered bandages or films.

In a second aspect, the present invention provides a transdermal drug delivery composition comprising (a) testosterone; (b) at least one volatile solvent; (c) a penetration enhancer which is one or a mixture of octyl dimethyl-para-aminobenzoate, octyl para-methoxycinnamate and octyl salicylate; and (d) at least one viscosity modulating agent, for use in the treatment of testosterone deficiency in a subject by applying the composition to at least one axilla to deliver testosterone systemically; wherein the transdermal drug delivery composition is for application without being covered by adhesives, adhered patches, adhered bandages or films.

In one embodiment of the first and second aspects, the composition is for the treatment of testosterone deficiency in men, such as for treatment of androgen deficiency in adult males. In one embodiment of the first and second aspects, the penetration enhancer is present in an amount of 0.01 to 15% of the total composition. In one embodiment of the first and second aspects, the volatile solvent is a lower alkyl alcohol or mixture of such alcohols. In particular, the volatile solvent may have a vapour pressure above 35 mm Hg at atmospheric pressure and at a temperature of 32°C. For example, the volatile solvent may be selected from the group consisting of ethanol, ethyl acetate, isopropanol, acetone, ethyl formate, methyl acetate, methyl ethyl ketone, pentane and chloroform or mixtures thereof in the range of 40 to 99% v/v of the composition. In one embodiment of the first and second aspects, the volatile solvent is present at 80% w/w or more. In one embodiment of the first and second aspects, the composition comprises over 80% alcohol and the viscosity modulating agent is polyvinylpyrrolidone (PVP) in an amount of from 1% to 3%. In one embodiment of the first and second aspects, the composition is applied no more than once a day. In one embodiment of the first and second aspects, the composition has a viscosity of less than 300 centipoise. In one embodiment of the first and second aspects, the composition is for achieving a testosterone blood level of at least 200 ng/dL.

The applicant has developed a method and a composition for transdermal administration which allows rapid delivery of active agents and reduces the risk of undesirable side effects in a patient. The delivery is "rapid" in the sense that little time of the patient is required. For example, in one embodiment, the transdermal composition is dry within 3 minutes. Pharmacodynamically, delivery of the active is substantially "steady-state", once a reservoir of the active is established in the skin. The reservoir is maintained (ie, "topped-up") by daily doses of the composition. Steady-state is the practical description of the delivery profile - there is some, but surprisingly minor, variation in delivery rates between doses. The composition therefore
allows rapid delivery of active agents and minimises the risk of side effects in a patient.

The applicant has surprisingly observed that the transdermal composition used in the current invention has antiperspirant and/or deodorant properties. The composition therefore allows the active agent to be rapidly delivered whilst also enabling perspiration and/or odour to be reduced. The use of the invention thus permits use of the axilla for transdermal drug delivery without patient inconvenience of not using normal antiperspirant/deodorant products (important for patient compliance) which may otherwise interfere with delivery of the active.

Preferably the viscosity modulating agent is a gelling or thickening agent. The viscosity modulating agent may be a thickening agent and suitable thickening agents include polyvinyl pyrrolidone. In some embodiments, the thickening agent is an antiperspirant or the composition further includes an antiperspirant and/or a deodorant.

In this specification and the claims, the term "viscosity modulating agent" is used to refer to a component of the composition which alters the viscosity of the overall resulting composition. The nature of the viscosity modulating agent depends not only on the agent itself, but also the proportion in which it is present and the presence or absence of other components. For example, a gelling agent may act as a viscosity modulating agent providing that an activator for that gelling agent is present. For example, hydroxypropylmethylcellulose (HPMC) may be used in a composition with an activator, in which the volatile solvent is a lower alkyl alcohol at a concentration of around 2% w/w. A suitable activator would be sodium chloride. Concentration may be important as, in this example, at 0.1% w/w HPMC has different effects. A thickening agent is one which increases viscosity, and is often anhydrous. Viscosity modulating agents are further described below.

Further, the term "transdermal" is used co-terminously with "topical" in describing the application of agents to the skin. Both terms "topical" and "transdermal" are used herein in the broadest sense to refer to administration of a drug to the skin surface of an animal, including humans, so that the drug passes through the skin tissue. Unless otherwise stated or implied, the terms topical drug delivery and transdermal drug delivery are used interchangeably. From a strict drug-delivery perspective, "transdermal" is sometimes used to refer only to systemic delivery through the skin whereas "topical" requires only delivery into or on the skin for local effect. The invention described in this specification is equally applicable to both transdermal and topical modes of delivery, and is described here as "transdermal" only for convenience.

Typically, the composition appears like a lotion. In this context, "lotion" is used in its broad descriptive sense, rather than the more specific formulatory sense which refers to a mixed phase or suspension of active. The composition used in the invention is often a true solution, but with increased viscosity so that its viscosity is more similar to that usually associated with a lotion. The viscosity of the composition is preferably greater than that of water but less than 300 centipoise. The viscosity in different embodiments is in the range of 10 to 200, 20 to 100 or 30 to 50 centipoise.

The penetration enhancer assists in the uptake of the physiologically active agent at least through the outer layers of the skin, typically to form a reservoir of active within the skin.

The volatile solvent (also sometimes called a "volatile carrier" or "vehicle") will typically be present in a higher concentration, such as 80% or more w/w. The volatile solvent may be any such solvent that is pharmacologically suitable and many such solvents are known in the art. One of the advantages of the inclusion of a volatile solvent or volatile carrier is that it facilitates the composition to dry rapidly, allow the absorption of the active agent, and avoid the problems of accidentally dosing others by confining administration to a small area of skin, preferably the axilla. Preferably the volatile solvent has a vapour pressure above 35mm Hg at atmospheric pressure and normal skin temperature of 32 degrees Celsius.

Preferably, the solvent is a lower alkyl alcohol or a mixture of such alcohols. Suitable solvents include ethanol, ethyl acetate, isopropanol, acetone, ethyl formate, methyl acetate, methyl ethyl ketone, pentane and chloroform or mixture thereof in the range of
40 to 99% v/v of the composition, preferably more than 50%, 60%, 70% or 80%. An aerosol propellant, such as dimethyl ether or R134a, may also constitute a volatile solvent for the purpose of the present invention.

Preferably the compositions used in the invention are non-occlusive, in that in the broadest sense, the composition is not trapped to the skin, or the skin is not closed to the atmosphere, by means of a patch device, fixed reservoir, application chamber, tape, bandage, sticking plaster, or the like, which remains on the skin a the site of application for a prolonged length of term. Such devices tend to be uncomfortable for the wearer or can be embarrassing or unsightly.

The viscosity modulating agent will commonly be a thickening agent or a gelling agent. It will often be used to increase the viscosity of the composition containing a solution of the physiologically active agent in the volatile solvent. Given the nature of the volatile solvents, the solution will typically have very low viscosity. The purpose of the viscosity modulating agent is to increase the viscosity of the solution such that the composition is retained in the vicinity of the area of application for a brief period of time so as to permit increased uptake of the physiologically active agent at that site. The viscosity modulating agent will typically increase the viscosity to about that of a typical lotion (eg, sunscreen), but not to the point where the composition becomes a gel. Typically, the viscosity of a transdermal drug delivery composition used according to the invention will be less than 300 centipoise and often about 150 centipoise.

The viscosity modulating agent must retain its activity in the context of the other components of the composition used in the invention. In particular, the thickening agent must remain active and stable in this environment. For example, where the composition has a high alcohol content (for example, where the volatile solvent comprises primarily alcohol at greater than 80% v/v), the thickening agent must be effective in a high alcoholic environment. Having these requirements in mind, a skilled person can select several thickening agents from those known in the art. Desirably, a thickening agent also inhibits the solvent evaporation rate so as to enhance the so-called "solvent burst" of active agent into the skin at the site of application.

It will be appreciated by one skilled in the art that the amount of thickening agent required is a question of degree and compromise with other parameters. It is also known that many thickening agents have peak activity at a particular concentration, and that activity may drop off substantially with slightly higher and slightly lower percentage concentrations. For example, in one preferred embodiment where the composition comprises over 80% alcohol and the thickening agent used is PVP, the desirable concentration of PVP is between 1 and 3%, and its activity is substantially reduced outside that range.

Gelling agents are matrix-forming agents which, once activated, act by forming a matrix within and around the composition they are in. Thickening agents are usually anhydrous agents which increase the viscosity of the composition. In one embodiment, a thickening agent is used. The thickening agent may be an antiperspirant and/or an occlusive agent for the drug delivery composition. In another embodiment, both a deodorant and an antiperspirant are in a composition with the at least one active agent and dermal penetration enhancer. Suitable thickening agents include polyvinyl pyrrolidone or PVP (Povidone™). The antiperspirant may be an occlusive agent also, and a thickening, occlusive agent may have antiperspirant effects.

Despite the inherent antiperspirant and/or deodorant properties of the compositions used in the invention, the composition may be optionally administered with deodorant and antiperspirant additives that do not interfere with the active.

The antiperspirant agent may be any suitable substance that reduces or inhibits the production of perspiration. In some instances, an antiperspirant agent can also provide deodorant benefits. Preferably, the antiperspirant agent is selected from the group consisting of inorganic or organic salts of aluminium, zirconium, zinc or mixtures thereof.

In one embodiment, the antiperspirant agent is an aluminium salt having the general formula:

Al₂(OH)ₓQ_{y}.wH₂O

where Q is chlorine, bromine or iodine;
x is 2 to 5;
x+y = 6, where x and y do not need to be integers; and
wH₂O represents a variable amount of hydration.

In another embodiment, the antiperspirant agent is a zirconium salt of the following general formula:

ZrO(OH)_{2n-nz}B_{z}.wH₂O

where
z is a variable in the range of from 0.9 to 2.0 so that the value of 2n-nz is zero or a positive;
n is the valency of B;
B is selected from the group consisting of chloride, other halide, sulphamate, sulphate and mixtures thereof; and
wH₂O represents a variable amount of hydration.

In a preferred embodiment, the antiperspirant agent is selected from the group consisting of aluminium chloride, aluminium chlorohydrate, aluminium chlorohydrex polyethylene glycol, aluminium chlorohydrex propylene glycol, aluminium dichlorohydrate, aluminium dichlorohydrex polyethylene glycol, aluminium dichlorohydrex propylene glycol, aluminium sesquichlorohydrate, aluminium sesquichlorohydrex polyethylene glycol, aluminium sesquichlorohydrex propylene glycol, aluminium zirconium octachlorohydrate, aluminium zirconium octachlorohydrex gly, aluminium zirconium pentachlorohydrate, aluminium zirconium pentachlorohydrex gly, aluminium zirconium tetrachlorohydrate, aluminium zirconium tetrachlorohydrex gly, aluminium zirconium trichlorohydrate and aluminium zirconium trichlorohydrex gly. These antiperspirant agents have approved listings under the United States Food & Drug Administration Federal Register.

The present invention also contemplates that other antiperspirant agents may also be used. Examples of these antiperspirant agents include aluminium bromohydrate, aluminium chloride, aluminium citrate, aluminium sulfate, ammonium alum, cobalt acetylmethionate, potassium alum, sodium alum and sodium aluminium chlorohydroxy lactate.

In another embodiment, the composition used in the invention comprises a deodorant agent. The deodorant agent may be any suitable substance that provides deodorancy benefits in masking or neutralising odours that are produced by the action of bacteria. Generally, deodorant agents do not interfere with the production of perspiration. Representative examples of deodorant agents include, but are not limited to, one or more of cetyl-trimethylammonium bromide, cetyl pyridinium chloride, benzethonium chloride, diisobutyl phenoxy ethoxy ethyl dimethyl benzyl ammonium chloride, sodium N-lauryl sarcosine, sodium N-palmithyl sarcosine, lauroyl sarcosine, N-myristoyl glycine, potassium N-lauryl sarcosine, stearyl, trimethyl ammonium chloride, sodium aluminium chlorohydroxy lactate, tricetylmethyl ammonium chloride, 2,4,4'-trichloro-2'-hydroxy diphenyl ether, diaminoalkyl amides such as L-lysine hexadecyl amide, heavy metal salts of citrate, salicylate, and piroctose, especially zinc salts, and acids thereof, heavy metal salts of pyrithione, especially zinc pyrithione and zinc phenolsulfate. Other deodorant agents include, without limitation, odour absorbing materials such as carbonate and bicarbonate salts, e.g. as the alkali metal carbonates and bicarbonates, ammonium and tetraalkylammonium carbonates and bicarbonates, especially the sodium and potassium salts, or any combination of the above.

In a preferred embodiment, the composition comprises a combination of antiperspirant and deodorant agents.

The antiperspirant and deodorant agents may be present in the composition in any amount that provides beneficial antiperspirant and/or deodorancy effects. The antiperspirant agent or deodorant agent may be present in an amount of from 0.05 to 60%, and is preferably from 1 to 40%, more preferably from 5 to 30% and even more preferably from 8 to 15% by weight of the composition. Where the composition used in the invention comprises a combination of antiperspirant and deodorant agents, the combined amounts of these agents is preferably within the preferred range stated above.

The composition is to be applied to at least one axilla. Preferably it is applied to further areas of skin having increased cutaneous blood flow, such as the scrotum, between toes, groin, plantar arch of the foot or palm of the hand. Other suitable areas include where there is suitably permeable skin. In particularly, it is desirable to apply it to only one or more of these areas while still effectively delivering the required dose of active agent. It is also known that some areas of the skin are relatively more permeable to active agents than others. The invention is usefully employed to deliver active agent through these areas only, thus limiting the total area of skin to which the composition must be applied in order to deliver an effective dose of the active agent.

Described herein is a method for transdermal delivery of an active agent to areas of the skin prone to perspiration, particularly higher rates of perspiration relative to other areas. Such areas of skin have typically not been preferred for transdermal delivery because of the desirability for patients to be able to maintain use of antiperspirants and deodorants. In a preferred form, the method provides for administration of a composition including the active agent to those areas of skin which provide maximal systemic absorption. It is believed that this is due, at least in part, to increased cutaneous blood flow and heat. These areas may be one of more of the axilla, scrotum, groin, planter arch of foot, palm of hand or forehead. Preferably the area is at least one axilla. These areas in particular may benefit greatly from the antiperspirant and/or deodorant effects of the composition when it is applied.

In another form, described herein is a method of delivering an active agent systemically to a patient by application of the active agent to one or more axilla. The active agent is preferably in a transdermal drug delivery composition as described in this specification. Preferably, administration is essentially only to the axilla. In another form, administration is to areas selected from the group consisting of the axilla, scrotum, groin, planter arch of foot, palm of hand and forehead. In one form, the method avoids application or administration of the composition to the upper arms, shoulders or abdomen of a subject. The transdermal drug delivery composition used in the present invention is capable, upon application to an area of skin, of delivering a therapeutically effective amount of active agent to the systemic circulation.

The composition may be applied to any area of skin on a subject, selected from one or more of planter foot arch, lateral ankle, palm, upper arm, ventral forearm, dorsal forearm, back, chest, thigh, abdomen, groin, scalp, axilla, forehead, lower back, buttocks or scrotum. The composition is preferably applied to those areas of skin which provide maximal systemic absorption due to increased cutaneous blood flow and heat. These areas may be one of more of the axilla, scrotum, planter arch of foot, palm of hand or forehead. Preferably the area is at least one axilla. These areas in particular may benefit greatly from the antiperspirant and/or deodorant effects of the composition when it is applied.

A further advantage of the compositions used according to the invention is that a therapeutically effective amount may be applied to a minimal surface area, therefore minimising the unpleasant side effects and patient inconvenience associated with applying large amounts of a composition to a large surface area. Preferably the area of skin to which the composition is applied is less than 500cm². Desirably, the area is less than 300cm², 100 cm², 50cm², 20cm² 10cm², and 5cm².

A key advantage achievable with some embodiments of the invention is increased flux of the active, thereby enabling a greater proportion of active in a dose to be delivered to the patient and utilising a smaller area of skin. In some cases, a much smaller area than used to date is required, consequently improving patient acceptability and compliance. This is particularly applicable where the active otherwise has poor or low flux, a large systemic dose is desired and/or a faster onset of action is desired.

The penetration enhancer is also sometimes called an "absorption" enhancer. A number of penetration enhancers are known including fatty acids, fatty acid esters, fatty alcohols, glycols and glycol esters, 1,3-dioxolanes and 1,3-dioxanes, macrocyclic ketones containing at least 12 carbon atoms, oxazolidinones and oxazolidinone derivatives, alkyl-2-(N,N-disubstituted amino)-alkanoate esters, (N,N-disubstituted amino)-alkanol alkanoates, sunscreen esters and mixtures thereof, such as those
including oleic acid, oleyl alcohol, cyclopentadecanone (CPE-218™), sorbitan monooleate, glycerol monooleate, propylene glycol monolaurate, polyethylene glycol monolaurate, 2-n-nonyl 1,3-dioxolane (SEPA™), dodecyl 2-(N,N-dimethylamino)-propionate (DDAIP) or its salt derivatives, 2-ethylhexyl 2-ethylhexanoate, isopropyl myristate, dimethyl isosorbide, 4-decyloxazolidinon-2-one (SR-38™,TCPI, Inc.), 3-methyl-4- decyloxazolidinon-2-one, octyl dimethyl-para-aminobenzoate, octyl para-methoxycinnamate, octisalate and mixtures thereof.

Some penetration enhancers are sunscreen esters such as the compounds disclosed in our US Patent No. 6,299,900.

These include the compounds being safe skin-tolerant ester sunscreens of formula: 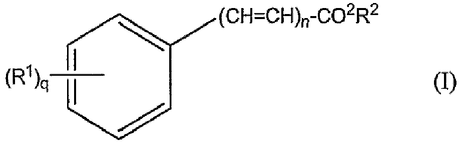
wherein R¹ is hydrogen, lower alkyl, lower alkoxy, halide, hydroxy or NR³R⁴;
R² is long chain alkyl;
R³ and R⁴ are each independently hydrogen, lower alkyl or R³ and R⁴ together with the nitrogen atom to which they are attached form a 5- or 6-membered heterocyclic ring;
n is 0 or 1; and
q is 1 or 2.

The penetration enhancer used in the present invention is one or a mixture of octyl dimethyl-paraaminobenzoate ("Padimate O"), octyl para-methoxycinnamate and octyl salicylate ("octisalate" per USP).

The concentration of absorption/penetration enhancer may be in the range from 10-10,000 weight percent of absorption/penetration enhancer based upon the weight of active ingredient. The ratio of penetration enhancer to active ingredient may vary considerably and will be governed as much as anything, by the pharmacological results that are required to be achieved. In principle, it is desirable that as little absorption enhancer as possible is used. However, usually the penetration enhancer is in the range from 0.01-15% of the total composition.

Heat can also act to enhance penetration of an active agent, together with or independently of such a penetration enhancer. Heat is not itself regarded as a "penetration enhancer". Without being bound by the theory or mode of action, it is believed that heat can disrupt and/or fluidise the lipid structures in the skin to enhance penetration.

Heat may result from utilising the patient's own body heat to warm the skin to the core body temperature (the outer layers of skin often being below that temperature) and/or the heat may be from an external source. Such external sources include conventional heat packs (eg, for therapeutic or physiotherapeutic uses), radiation heat (eg, heat lamps), exothermic chemical reactions or *in situ* generation of heat.

Heat is suggested to enhance the transdermal delivery of various drugs by increasing skin permeability, body fluid circulation, blood vessel wall permeability, rate-limiting membrane permeability, and drug solubility. Accordingly, whether or not heat is applied around the time of application of the composition, application of a transdermal composition is preferably to areas of skin having increased cutaneous blood flow, and therefore warmer. Such areas include but are not limited to, the planter arch of the foot, palm of the hand, axilla and scrotum. However associated with areas of increased heat is increased perspiration and perspiration related body odour. Therefore, it is desirable if the transdermal composition is also an antiperspirant and/or a deodorant. An antiperspirant reduces the amount of perspiration produced by blocking the sweat glands. A deodorant masks or covers the odour associated with perspiration, using either fragrance or an anti-bacterial ingredient to reduce odour-causing bacteria on the skin. Antiperspirants can have an indirect deodorant effect in the underarm area, but a deodorant cannot act as an antiperspirant. It is further desirable if known antiperspirants and/or deodorants can be added to the composition without inhibiting drug delivery.

Without being bound by any theory, the applicant believes that the viscosity modulating agent and/or penetration enhancer in the composition function to minimise perspiration, and that the volatile carrier functions to inhibit bacteria and therefore odour by a bactericidal activity of the carrier.

In one embodiment, the composition consists essentially of one physiologically active agent (i.e., testosterone); one volatile solvent; the penetration enhancer; and one viscosity modulating agent, each as described above. In one embodiment, the viscosity modulating agent is an antiperspirant, and the composition optionally also includes a deodorant. Each of these embodiments may or may not also include water.

In another embodiment of the invention, the composition may include at least one additional active agent and/or at least one additional inactive agent. In a different embodiment, the composition does not include a herbal extract (or like component), whether as a physiologically active agent or otherwise.

The transdermal drug delivery composition used in the present invention is capable, upon application to an area of skin, of delivering a therapeutically effective amount of active agent to the systemic circulation, e.g. of
delivering a predetermined amount of active and/or sufficient active to achieve a predetermined bloodstream level or concentration of the active. For some actives (eg, those with a short half-life) other measures of the amount of active delivered will be appropriate.

The composition may also be administered by other known topical techniques including a lotion, gel, spray etc. These can be formulated by adding suitable carriers, excipients and thixotropic agents which are inert to the active to facilitate retaining the composition on the skin sufficiently for delivery of the active agent as contemplated by the invention.

The transdermal composition used in the
invention may be applied, including to axilla, of a subject in any of a range of forms. Suitable forms include for example lotions, gels, foams, pastes, soft solids, firm sticks, solutions, sprays, aerosols, roll-ons and the like, each of which represent different forms of the invention. The composition may be applied in an occlusive or non-occlusive manner. It is preferred that the composition is applied in a non-occlusive manner and in the most preferred embodiment the composition is applied as a lotion, aerosol or spray.

The transdermal composition used in the
invention may further comprise additional components that will facilitate its preparation into forms suitable for application to the axilla of a subject. Examples of additional components include but are not limited to surfactants, buffers, solvents and propellants.

The composition used
in the invention is applied without being covered by adhesives, adhered patches, adhered bandages or films. Such devices tend to be uncomfortable for the wearer or can be embarrassing or unsightly. The invention allows non-occlusive administration of physiologically active agents by the transdermal compositions in the axilla under the subjects clothing.

In applying the composition used in the invention, an antiperspirant and/or a deodorant may be applied at about the same time as the composition. Preferably, the antiperspirant and/or deodorant is applied after application of the composition. As noted above, in some embodiments of the invention, an antiperspirant and/or a deodorant may be incorporated within the composition, thus requiring only one substance to be applied by a patient. The antiperspirant and/or deodorant will be selected from those which do not interfere with the drug delivery mechanism. Preferably, the antiperspirant and deodorant added complement the natural antiperspirant and deodorising properties of the transdermal drug delivery composition itself as described above.

In one embodiment,
an antiperspirant and/or a deodorant is applied to the axilla region at about the same time as the transdermal drug delivery composition is applied. Preferably, the antiperspirant and/or deodorant is applied after the transdermal drug delivery composition is applied to at least one axilla region.

The compositions are preferably administered without being covered by an adhesive bandage, patch or other physical barrier bonded to the administration area. Preferably the transdermal composition becomes touch dry within three minutes of application to the area of skin, more preferably within about one minute.

The antiperspirant and/or deodorant properties of the composition are particularly advantageous when the physiologically active agent is testosterone or derivatives thereof for treating testosterone deficiency in a subject. Testosterone is responsible for increasing perspiration and producing perspiration and perspiration related odour in the presence of 5-alpha-reductase, due to the conversion of testosterone to dihydrotestosterone (DHT). Accordingly disclosed herein is a composition as described above in which the physiologically active agent is selected from at least one or more of androgenic compound, such as testosterone or derivatives thereof. The physiologically active agent may therefore be selected from androgenic compounds which are synthetically derivatized from testosterone and are known to provide the same or a similar physiological activity. Such compounds include without limitation, testosterone salts, such as acetate, enanthate, cypionate, isobutyrate, dehydro-epiandrosterone, propionate, undecanoate esters and cyproterone acetate. In other embodiments, one or more of the following are included: 5-alpha-reductase inhibitors (such as finasteride, turosteride, LY-191704 and MK-386). Other suitable compounds include methyltestosterone, clostebol acetate, drostanolone, furazabol, nandrolone oxandrolone, stanozolol, trenbolone acetate, dihydro-testosterone, 17-.alpha.-methyl-19-nortestosterone and fluoxymesterone.

Contrary to what might be expected from the activity of testosterone, the applicant has found that testosterone may be administered in accordance with the invention to provide rapid delivery without inducing sweating and odour that distress a subject. The unacceptable levels of these side effects identified in the art have been attributed to the presence of elevated testosterone.

In one embodiment, the volatile carrier is isopropyl alcohol, the penetration enhancer is octisalate, and the thickening agent is polyvinylpyrrolidone. These may be in the following percentages:
30% v/v volatile carrier;
8% w/v penetration enhancer;
1% w/v active (i.e., testosterone);
2% w/v thickener;
10% v/v sterile water; and
the balance ethanol.

In another embodiment, the transdermal drug delivery composition consists essentially of the above 6 components in those proportions. In another form of the invention, these 6 components in these proportions are used in the manufacture of a medicament for the treatment of a subject, in particular
a subject suffering from decreased testosterone blood levels.

In one embodiment,the composition containing testosterone as the active agent is applied to the axilla of a patient to deliver testosterone resulting in a blood level of at least a predetermined amount. In one embodiment, the predetermined amount is the normal range. In the case of testosterone, the blood level achieved is at least 200 ng/dL, preferably 300-1000 ng/dL. Preferably, the composition is only applied to the axilla.

In another embodiment, the composition may further include a second active agent to provide the composition with additional usage benefits. The second active agent may be any one of those listed below, or herbal extracts and/or cosmetic agents (such as, age spot and keratose removing agents, anti-aging agents, antioxidants, and hydroxy acids).

Physiologically active agents that may be used in the transdermal drug delivery compositions used in the present invention include any locally or systemically active agents which are compatible with the penetration enhancers used in the present invention and which can be delivered through the skin, particularly with the assistance of the dermal penetration enhancer to achieve a desired effect. These active agents are preferably therapeutics, and include (grouped by therapeutic class):
(a) Alimentary System agents including antidiarrhoeals such as diphenoxylate, loperamide and hyoscyamine.
(b) Cardiovascular System agents including:
   (i) antihypertensives such as hydralazine, minoxidil, captopril, enalapril, clonidine, prazosin, debrisoquine, diazoxide, guanethidine, methyldopa, reserpine, trimetaphan;
   (ii) Calcium channel blockers such as diltiazem, felodopine, amlodipine, nitrendipine, nifedipine and verapamil;
   (iii) antiarrhythmics such as amiodarone, flecainide, disopyramide, procainamide, mexiletene and quinidine;
   (iv) antiangina agents such as glyceryl trinitrate, erythritol tetranitrate, pentaerythritol tetranitrate, mannitol hexanitrate, perhexilene, isosorbide dinitrate and nicorandil;
   (v) beta-adrenergic blocking agents such as alprenolol, atenolol, bupranolol, carteolol, labetalol, metoprolol, nadolol, nadoxolol, oxprenolol, pindolol, propranolol, sotalol, timolol and timolol maleate;
   (vi) cardiotonic glycosides such as digoxin and other cardiac glycosides and theophylline derivatives;
   (vii) adrenergic stimulants such as adrenaline, ephedrine, fenoterol, isoprenaline, orciprenaline, rimeterol, salbutamol, salmeterol, terbutaline, dobutamine, phenylephrine, phenylpropanolamine, pseudoephedrine and dopamine;
   (viii) vasodilators such as cyclandelate, isoxsuprine, papaverine, dipyrimadole, isosorbide dinitrate, phentolamine, nicotinyl alcohol, co-dergocrine, nicotinic acid, glyceryl trinitrate, pentaerythritol tetranitrate and xanthinol; and
   (ix) antimigraine preparations such as ergotamine, dihydroergotamine, methysergide, pizotifen and sumatriptan.
(c) Drugs Affecting Blood and Haemopoietic Tissues including:
   (i) Anticoagulants and thrombolytic agents such as warfarin, dicoumarol, low molecular weight heparins such as enoxaparin; streptokinase and its active derivatives; and
   (ii) haemostatic agents such as aprotinin, tranexamic acid and protamine.
(d) Drugs Affecting the Central Nervous System including:
   (i) Analgesics;
   (ii) antipyretics including the opioid analgesics such as buprenorphine, dextromoramide, dextropropoxyphene, fentanyl, alfentanil, sufentanil, hydromorphone, methadone, morphine, oxycodone, papaveretum, pentazocine, pethidine, phenoperidine, codeine and dihydrocodeine; and
   (iii) Others include acetylsalicylic acid (aspirin), paracetamol, and phenazone.
(e) Hypnotics and sedatives such as the barbiturates, amylobarbitone, butobarbitone and pentobarbitone and other hypnotics and sedatives such as choral hydrate, chlormethiazole, hydroxyzine and meprobamate.
(f) Antianxiety agents such as the benzodiazepines, alprazolam, bromazepam, chlordiazepoxide, clobazam, chlorazepate, diazepam, flunitrazepam, filurazepam, lorazepam, nitrazepam, oxazepam, temazepam and triazolam.
(g) Neuroleptic and antipsychotic drugs such as the phenothiazines, chlorpromazine, fluphenazine, pericyazine, perphenazine, promazine, thiopropazate, thioridazine and trifluoperazine and the butyrophenones, droperidol and haloperidol and the other antipsychotic drugs such as pimozide, thiothixene and lithium.
(h) Antidepressants such as the tricyclic antidepressants amitryptyline, clomipramine, desipramine, dothiepin, doxepin, imipramine, nortriptyline, opipramol, protriptyline and trimipramine and the tetracyclic antidepressants such as mianserin and the monoamine oxidase inhibitors such as isocarboxazid, phenelizine, tranylcypromine and moclobemide and selective serotonin re-uptake inhibitors such as fluoxetine, paroxetine, citalopram, fluvoxamine and sertraline.
(i) CNS stimulants such as caffeine.
(j) Anti-alzheimer's agents such as tacrine.
(k) Anti-parkinson agents such as amantadine, benserazide, carbidopa, levodopa, benztropine, biperiden, benzhexol, procyclidine and dopamine-2 agonists such as S(-)-2-(N-propyl-N-2-thienylethylamino)-5-hydroxytetralin (N-0923).
(I) Anticonvulsants such as phenytoin, valproic acid, primidone, phenobarbitone, methylphenobarbitone and carbamazepine, ethosuximide, methsuximide, phensuximide, sulthiame and clonazepam.
(m) Antiemetics, antinauseants such as the phenothiazines, prochloperazine, thiethylperazine and 5HT-3 receptor antagonists such as ondansetron and granisetron and others such as dimenhydrinate, diphenhydramine, metoclopramide, domperidone, hyoscine, hyoscine hydrobromide, hyoscine hydrochloride, clebopride and brompride.
(n) Musculoskeletal System including:
   (i) Non-steroidal anti-inflammatory agents including their racemic mixtures or individual enantiomers where applicable, such as ibuprofen, flurbiprofen, ketoprofen, aclofenac, diclofenac, aloxiprin, aproxen, aspirin, diflunisal, fenoprofen, indomethacin, mefenamic acid, naproxen, phenylbutazone, piroxicam, salicylamide, salicylic acid, sulindac, desoxysulindac, tenoxicam, tramadol and ketoralac;
   (ii) Additional non-steroidal antiinflammatory agents which can be formulated in combination with the dermal penetration enhancers include salicylamide, salicylic acid, flufenisal, salsalate, triethanolamine salicylate, aminopyrine, antipyrine, oxyphenbutazone, apazone, cintazone, flufenamic acid, clonixeril, clonixin, meclofenamic acid, flunixin, coichicine, demecolcine, allopurinol, oxypurinol, benzydamine hydrochloride, dimefadane, indoxole, intrazole, mimbane hydrochloride, paranylene hydrochloride, tetrydamine, benzindopyrine hydrochloride, fluprofen, ibufenac, naproxol, fenbufen, cinchophen, diflumidone sodium, fenamole, flutiazin, metazamide, letimide hydrochloride, nexeridine hydrochloride, octazamide, molinazole, neocinchophen, nimazole, proxazole citrate, tesicam, tesimide, tolmetin, and triflumidate;
   (iii) Antirheumatoid agents such as penicillamine, aurothioglucose, sodium aurothiomalate, methotrexate and auranofin;
   (iv) Muscle relaxants such as baclofen, diazepam, cyclobenzaprine hydrochloride, dantrolene, methocarbamol, orphenadrine and quinine; and
   (v) Agents used in gout and hyperuricaemia such as allopurinol, colchicine, probenecid and sulphinpyrazone.
(o) Hormones and Steroids including:
   (i) Oestrogens such as oestradiol, oestriol, oestrone, ethinyloestradiol, mestranol, stilboestrol, dienoestrol, epioestriol, estropipate and zeranol;
   (ii) Progesterone and other progestagens such as allyloestrenol, dydrgesterone, lynoestrenol, norgestrel, norethyndrel, eclometrine, norethisterone, norethisterone acetate, gestodene, levonorgestrel, medroxyprogesterone and megestrol;
   (iii) Antiandrogens such as cyproterone acetate and danazol;
   (iv) Antioestrogens such as tamoxifen and epitiostanol and the aromatase inhibitors, exemestane and 4-hydroxy-androstenedione and its derivatives. Androgens and anabolic agents such as methyltestosterone, clostebol acetate, drostanolone, furazabol, nandrolone oxandrolone, stanozolol, trenbolone acetate, dihydro-testosterone, 17-.alpha.-methyl-19-nortestosterone and fluoxymesterone;
   (v) 5-alpha reductase inhibitors such as finasteride, turosteride, LY-191704 and MK-386;
   (vi) Corticosteroids such as betamethasone, betamethasone valerate, cortisone, dexamethasone, dexamethasone 21-phosphate, fludrocortisone, flumethasone, fluocinonide, fluocinonide desonide, fluocinolone, fluocinolone acetonide, fluocortolone, halcinonide, halopredone, hydrocortisone, hydrocortisone 17-valerate, hydrocortisone 17-butyrate, hydrocortisone 21-acetate methylprednisolone, prednisolone, prednisolone 21-phosphate, prednisone, triamcinolone, triamcinolone acetonide;
   (vii) Further steroidal antiinflammatory agents such as cortodoxone, fluoracetonide, fludrocortisone, difluorsone diacetate, flurandrenolone acetonide, medrysone, amcinafel, amcinafide, betamethasone and its other esters, chloroprednisone, clorcortelone, descinolone, desonide, dichlorisone, difluprednate, flucloronide, flumethasone, flunisolide, flucortolone, fluorornethalone, fluperolone, fluprednisolone, meprednisone, methylmeprednisolone, paramethasone, cortisone acetate, hydrocortisone cyclopentylpropionate, cortodoxone, flucetonide, fludrocortisone acetate, flurandrenolone acetonide, medrysone, amcinafal, amcinafide, betamethasone, betamethasone benzoate, chloroprednisone acetate, clocortolone acetate, descinolone acetonide, desoximetasone, dichlorisone acetate, difluprednate, flucloronide, flumethasone pivalate, flunisolide acetate, fluperolone acetate, fluprednisolone valerate, paramethasone acetate, prednisolamate, prednival, triamcinolone hexacetonide, cortivazol, formocortal and nivazol;
   (viii) Pituitary hormones and their active derivatives or analogs such as corticotrophin, thyrotropin, follicle stimulating hormone (FSH), luteinising hormone (LH) and gonadotrophin releasing hormone (GnRH);
   (ix) Thyroid hormones such as calcitonin, thyroxine and liothyronine and antithyroid agents such as carbimazole and propylthiouracil; and
   (x) Other miscellaneous hormone agents such as octreotide.
(q) Pituitary inhibitors such as bromocriptine.
(r) Ovulation inducers such as clomiphene.
(s) Hypoglycaemic agents such as insulin, chlorpropamide, glibenclamide, gliclazide, glipizide, tolazamide, tolbutamide, rosiglitazone and metformin.
(t) Genitourinary System agents.
(u) Diuretics such as the thiazides, related diuretics and loop diuretics, bendrofluazide, chlorothiazide, chlorthalidone, dopamine, cyclopenthiazide, hydrochlorothiazide, indapamide, mefruside, methycholthiazide, metolazone, quinethazone, bumetanide, ethacrynic acid and frusemide and potassium sparing diuretics, spironolactone, amiloride and triamterene.
(v) Antidiuretics such as desmopressin, lypressin and vasopressin including their active derivatives or analogs.
(w) Obstetric drugs including agents acting on the uterus such as ergometrine, oxytocin and gemeprost.
(x) Prostaglandins such as alprostadil (PGE1), prostacyclin (PGI2), dinoprost (prostaglandin F2-alpha) and misoprostol.
(y) Antimicrobials including:
   (i) cephalosporins such as cephalexin, cefoxytin and cephalothin;
   (ii) penicillins such as amoxycillin, amoxycillin with clavulanic acid, ampicillin, bacampicillin, benzathine penicillin, benzylpenicillin, carbenicillin, cloxacillin, methicillin, phenethicillin, phenoxymethylpenicillin, flucloxacillin, mezlocillin, piperacillin, ticarcillin and azlocillin;
   (iii) tetracyclines such as minocycline, chlortetracycline, tetracycline, demeclocycline, doxycycline, methacycline and oxytetracycline and other tetracycline-type antibiotics;
   (iv) minoglycosides such as amikacin, gentamicin, kanamycin, neomycin, netilmicin and tobramycin. Antifungals such as amorolfine, isoconazole, clotrimazole, econazole, miconazole, nystatin, terbinafine, bifonazole, amphotericin, griseofulvin, ketoconazole, fluconazole and flucytosine, salicylic acid, fezatione, ticlatone, tolnaftate, triacetin, zinc, pyrithione and sodium pyrithione;
   (v) Quinolones such as nalidixic acid, cinoxacin, ciprofloxacin, enoxacin and norfloxacin. Sulphonamides such as phthalylsulphthiazole, suffadoxine, sulphadiazine, sulphamethizole and sulphamethoxazole;
   (vi) Sulphones such as dapsone; and
   (vii) Other miscellaneous antibiotics such as chloramphenicol, clindamycin, erythromycin, erythromycin ethyl carbonate, erythromycin estolate, erythromycin glucepate, erythromycin ethylsuccinate, erythromycin lactobionate, roxithromycin, lincomycin, natamycin, nitrofurantoin, spectinomycin, vancomycin, aztreonam, colistin IV, metronidazole, tinidazole, fusidic acid and trimethoprim; 2-thiopyridine N-oxide; halogen compounds, particularly iodine and iodine compounds such as iodine-PVP complex and diiodohydroxyquin; hexachlorophene; chlorhexidine; chloroamine compounds; benzoylperoxide
(z) Anti-tuberculosis drugs such as ethambutol, isoniazid, pyrazinamide, rifampicin and clofazimine. Antimalarials such as primaquine, pyrimethamine, chloroquine, hydroxychloroquine, quinine, mefloquine and halofantrine.
(aa) Antiviral agents such as acyclovir and acyclovir prodrugs, famciclovir, zidovudine, didanosine, stavudine, lamivudine, zalcitabine, saquinavir, indinavir, ritonavir, n-docosanol, tromantadine and idoxuridine.
(ab) Anthelmintics such as mebendazole, thiabendazole, niclosamide, praziquantel, pyrantel embonate and diethylcarbamazine.
(ac) Cytotoxic agents such as plicamycin, cyclophosphamide, dacarbazine, fluorouracil and its prodrugs [described, for example, in International Journal of Pharmaceutics 111, 223-233 (1994)], methotrexate, procarbazine, 6-mercaptopurine and mucophenolic acid.
(ad) Metabolism agents including anorectic and weight reducing agents such as dexfenfluramine, fenfluramine, diethylpropion, mazindol and phentermine.
(ae) Agents used in hypercalcaemia such as calcitriol, dihydrotachysterol and their active derivatives or analogs.
(af) Respiratory System agents including:
   (i) antitussives such as ethylmorphine, dextromethorphan and pholcodine;
   (ii) expectorants such as acetylcysteine, bromhexine, emetine, guaiphenesin, ipecacuanha and saponins;
   (iii) decongestants such as phenylephrine, phenylpropanolamine and pseudoephedrine; and
   (iv) bronchospasm relaxants such as ephedrine, fenoterol, orciprenaline, rimiterol, salbutamol, tulobuterol, sodium cromoglycate, cromoglycic acid and its prodrugs [described, for example, in International Journal of Pharmaceutics 7, 63-75 (1980)], terbutaline, ipratropium bromide, salmeterol and theophylline and theophylline derivatives.
(ag) Allergy and Immune System agents including:
   (i) antihistamines such as meciozine, cyclizine, chlorcyclizine, hydroxyzine, brompheniramine, chlorpheniramine, clemastine, cyproheptadine, dexchlorpheniramine, diphenhydramine, diphenylamine, doxylamine, mebhydrolin, pheniramine, tripolidine, azatadine, diphenylpyraline, methdilazine, terfenadine, astemizole, loratidine and cetirizine.
(ah) Local anaesthetics such as bupivacaine, amethocaine, lignocaine, cinchocaine, dibucaine, mepivacaine, prilocaine and etidocaine.
(ai) Stratum corneum lipids, such as ceramides, cholesterol and free fatty acids, for improved skin barrier repair [Man, et al. J. Invest. Dermatol., 106(5), 1096, 1996].
(aj) Neuromuscular blocking agents such as suxamethonium, alcuronium, pancuronium, atracurium, gallamine, tubocurarine and vecuronium.
(ak) Smoking cessation agents such as nicotine, bupropion and ibogaine.
(al) Insecticides and other pesticides which are suitable for local or systemic application.
(am) Dermatological agents, such as vitamins A and E, vitamin E acetate and vitamin E sorbate.
(an) Allergens for desensitisation such as house dust mite allergen.
(ao) Nutritional agents, such as vitamins, essential amino acids and essential fats.
(ap) Keratolytics such as the alpha-hydroxy acids, glycollic acid and salicylic acid.
(aq) Psychicenergisers, such as 3-(2-aminopropyl)indole, 3-(2-aminobutyl)indole, and the like.
(ar) Anti-acne agents such as containing isotretinoin, tretinoin and benzoyl peroxide.
(as) Anti-psoriasis agents such as containing etretinate, cyclosporin and calcipotriol.
(at) Anti-itch agents such as capsaicin and its derivatives such as nonivamide [Tsai, et al. Drug. Dev. Ind. Pharm., 20(4), 719, 1994].
(au) Anticholinergic agents, which are effective for the inhibition of axillary sweating and for the control of prickly heat. The antiperspirant activity of agents such as methatropine nitrate, propantheline bromide, scopolamine, methscopolamine bromide, and the new class of soft antiperspirants, quaternary acyloxymethyl ammonium salts [described, for example, by Bodor et al, J. Med. chem. 23, 474 (1980) and also in United Kingdom Specification No. 2010270, published Jun. 27, 1979].
(av) Other physiologically active peptides and proteins, small to medium-sized peptides, e.g., vasopressin and human growth hormone.

Preferably the active agents are androgens; oestrogens, preferably estradiol; progesterone and other progestagens; broncho-dilators; antianxiety agents, preferably buspirone; and central nervous system agents, preferably fentanyl.

Preferably the second active agent is an antifungal agent. Fungal infections are common in areas of the body having higher production of heat and perspiration.

In yet another embodiment, the composition may further comprise one or more inactive agents. Such inactive ingredients may be referred to as "additives". Examples of such additives include but are not limited to, humectants, deodorant agents, antiperspirants, pH adjusting agents, preservatives, emulsifiers, occlusive agents (including without limitation patches and film formers), solubilizing agents, colorants, and surfactants (including without limitation anionic surfactants).

The composition is
used to treat testosterone deficiency in men and women and the conditions and diseases resulting therefrom.

There are number of closely related androgenic compounds which are synthetically derivatized from testosterone are known to provide the same or a similar physiologic activity, as discussed above.

Testosterone production in both men and women declines naturally with age. Testosterone deficiency may result from disease or damage to the hypothalamus, pituitary gland, or testicles that inhibits hormone secretion and testosterone production, and is also known as hypogonadism. Depending on age, insufficient testosterone production can lead to abnormalities in muscle and bone development, underdeveloped genitalia, and diminished virility, libido and/or desire.

Testosterone deficiency in men (hypogonadism) may be present at birth (congenital) or may develop later (acquired). It is classified by the location of its cause along the hypothalamic-pituitary-gonadal axis:
- Primary, disruption in the testicles
- Secondary, disruption in the pituitary
- Tertiary, disruption in the hypothalamus.

The most common congenital cause is Klinefelter's syndrome. This condition, which is caused by an extra X chromosome, results in infertility, sparse facial and body hair, abnormal breast enlargement (gynecomastia), and small testes.

Congenital hormonal disorders such as leutenizing hormone-releasing hormone (LHRH) deficiency and gonadotropin-releasing hormone (GnRH) deficiency (e.g., Kallmann's syndrome) also may cause testosterone deficiency.

Other congenital causes include absence of the testes (anorchism; also may be acquired) and failure of the testicles to descend into the scrotum (cryptorchidism).

Acquired causes of testosterone deficiency include chemotherapy; damage occurring during surgery involving the pituitary gland, hypothalamus, or testes; glandular malformation; head trauma that affects the hypothalamus; infection (e.g., meningitis, syphilis, mumps); isolated LH deficiency (e.g., fertile eunuch syndrome); radiation; testicular trauma; and tumours of the pituitary gland, hypothalamus, or testicles.

Androgen deficiency in women has been associated with an increased rate of sexual problems or complaints in a number of studies. These problems are frequently encountered in oophorectomized women and those with androgen deficiency from other causes. Hypoactive sexual desire disorder (HSDD) in women is the persistent or recurring deficiency (or absence) of sexual fantasies, thoughts and/or desire for, or receptivity to, sexual activity, which causes personal distress. The cause may be either physiological or psychological or a combination of both. Common physiological etiologies include hormone deficiencies, medications, and surgical interventions. Any disruption of the female hormonal milieu caused by these etiologies can result in decreased sexual desire. The lack of, or a decrease in, sexual desire may also be secondary to poor sexual arousal and response, or to pain associated with sexual activity. Another factor may be difficulty with inability to attain or maintain sufficient sexual excitement, a condition known as female sexual arousal disorder (FSAD).

The composition disclosed herein may also be used in treatment of sexual dysfunction in men and women.

Normal daily production of testosterone in normal young men ranges from 3-10 mg per day with diurnal variation (maximum ∼7am declining throughout the day). The aim of testosterone therapy in men is to deliver physiologic amounts of testosterone to the systemic circulation producing serum testosterone levels within the normal range for healthy men (e.g. 300-1000 ng/dL or 10-35 nM).

Several clinical studies have demonstrated that in conditions such as female sexual dysfunction, testosterone administration, which is aimed at restoring testosterone levels to normal reproductive levels, is effective in improving sexual function. The studies to date suggest that systemic administration of doses ranging from 150µg to 300µg a day would be sufficient to return testosterone levels to mid- to high premenopausal levels in androgen deficient women.

The invention may be used in treating a wide variety of conditions responsive to testosterone therapy such as hypogonadism (primary and secondary), AIDS Wasting Syndrome, micropenis, somatopause, andropause, viropause, or androgen deficiency in adult males (ADAM), anaemia from renal dialysis or chronic kidney disease, benign prostatic hyperplasia, acne, diabetes, infertility, libido, periodontal disease, post-anabolic steroid abuse, dry eyes, diabetic retinopathy, retinopathy, and Lupus Erythematosis decreased bone density (i.e. osteoporosis), hyperlipemia, predisposition toward prostrate cancer, heart disease, angina, and hypertension.

The composition used in the invention includes a volatile solvent; thus, one of the significant advantages of the composition is that it dries rapidly, allows absorption of the active agent (i.e. testosterone), and avoids the problems of accidentally dosing others by confining administration to axilla. The transdermal composition used in accordance with the invention does not interfere with the application and use of other substances or products on the skin of a subject.

In the most preferred embodiment, the transdermal composition
used in the invention is applied as a lotion, spray or aerosol which is formulated to dry on the skin within three minutes of application. In this way we have found that the composition is driven into the skin and the testosterone composition forms a reservoir in the skin which we have found is particularly active in enhancing blood levels via the axilla without the undesirable effects associated with high localised subcutaneous testosterone levels in this region.

The compositions used in the invention preferably have a drying time of less than three minutes. Drying time may be determined by *in vitro* or *in vivo* tests. A suitable in vitro test involves placing a 10 µL sample on a clean glass slide at room temperature (approx. 20°C) and using a four decimal place analytical balance the time take for the vehicle to stop evaporating is measured. The resulting drying times from three repetitions of the test may be averaged.

For *in vivo* drying time measurement 10 µLs applied to volar forearms (32°C) of three subjects and the drying time is measured by touch and visual verification (no visible surface vehicle or shine).

The invention will now be described with reference to the following examples. It is to be understood that the examples are provided by way of illustration of the invention and that they are in no way limiting to the invention.

### Examples

The examples are described with reference to the drawings. In the drawings:
Figure 1 is a graph showing the variation in blood level of testosterone with time after transdermal application;
Figure 2 is a graph of testosterone permeation profiles obtained after application of a deodorant spray to a composition according to one embodiment of the invention;
Figure 3 is a graph of testosterone permeation profiles obtained after application of ethanol (3 µL) to the same embodiment of the invention as in Figure 2;
Figure 4 is a graph of the testosterone permeation profiles obtained from the application of a different embodiment of the invention compared with application of a control;
Figure 5 is a graph of the estradiol permeation profile obtained from the application of another embodiment of the invention compared with application of a control.

### Example 1

This example compares the single dose pharmacokinetics of testosterone following application of a single dose of a metered dose of a testosterone lotion to (a) the inner arm and (b) axilla, in healthy women.

In this example, the composition (referred to here as "Composition 1") contained the following components in the amounts by weight specified.

**Table 1**

| **Component** | **Use** | **Concentration** |
|---|---|---|
| Testosterone USP | Active | 1% w/v% |
| Octisalate USP | Penetration enhancer | 8% w/v |
| Povidone USP | Thickener | 1-5 w/v% |
| Purified water USP | Vehicle | 10% v/v |
| Isopropyl alcohol USP | Vehicle | 30% v/v |
| Alcohol USP | Vehicle | balance |

An open label, two period study was conducted in 12 healthy premenopausal women.

In each period, 1 mL of a metered dose testosterone lotion was applied as a single dose to either the inner arm or the axilla according to the randomisation schedule. Samples were collected over 72 hours for each subject and subsequently analysed for testosterone content.

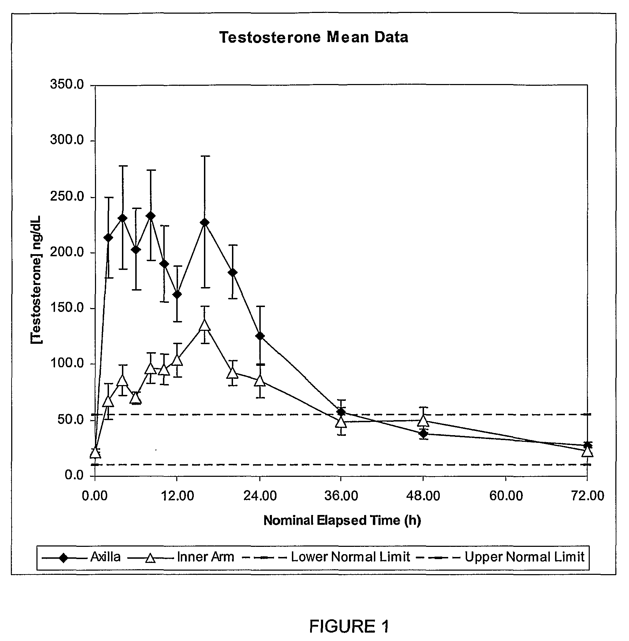

Two women were withdrawn from the study prior to the first dosing period. Ten women completed the study and the results are presented in the graph shown in Figure 1. The baseline corrected data comparing testosterone uptake from the axilla and the inner arm shows that the axilla has roughly a twofold increased uptake over the inner arm (AUC₀₋₇₂ was 5610.34 ng/dL/hr for the axilla compared to 2975.08 ng/dL/hr for the inner arm). No adverse sweating or odour was reported.

### Example 2

This example investigated the cumulative testosterone permeation through human skin *in vitro* following deodorant spray application.

Finite-dose *in vitro* diffusion studies were undertaken using excised human, male abdominal skin whereby the deodorant was applied to the skin surface at predetermined times after the topical application of a 5µL dose of a testosterone lotion, formulated according to example 1. These experiments were performed over 24 hours using stainless steel, flow through diffusion cells based on those described previously (Cooper, E.R. J. Pharm. Sci. 1984, 73, 1153-1156) except that the cell was modified to increase the diffusion area to 1.0cm². The formulations were applied using a finite dose technique (Franz, T.J. Curr. Probl. Dermatol., 1978, 7, 58-68) to mimic clinical dosing conditions at an applied dose volume of 5µL /cm². A piece of stainless steel wire mesh was placed directly below the skin in the receptor chamber of the of the diffusion cell to maintain a turbulent flow of receptor solution below the skin. The diffusion cells were maintained at a flow rate of approximately 1.0 mL/ cm²/hr by a microcassette peristaltic pump (Watson Marlow 505S UK). The cells were kept at 32 ± 0.5°C by a heater bar and the samples were collected into appropriately sized plastic vials on an automated fraction collector (Isco Retriever II, Lincoln, NE) at specified intervals. The receptor solution (20%v/v EtOH in 0.002% w/v NaN₃) maintained sink conditions below the skin.

At designated, pre-determined time points following the application of the testosterone lotion, the following were applied:
- one spray of a deodorant (containing isobutane, denatured alcohol, propane, triethyl citrate, parfum, butane, and water) was applied to the skin surface for approximately 1 sec from a constant distance of ∼10 cm from the top of the donor compartment of the cell; or
- 3 µL of ethanol (the estimated amount of ethanol present in a 1 sec spray of deodorant).

The amount of testosterone that permeated the skin was quantified using a validated HPLC method.

The application of a spray of deodorant at different time points after the topical application of the testosterone lotion did not have a significant effect (enhancing or inhibitory) on the permeation of testosterone through human epidermis *in vitro.* Application of neat ethanol (EtOH) after testosterone lotion dosing also revealed no significant effect on the permeation of testosterone lotion through human epidermis *in vitro.*

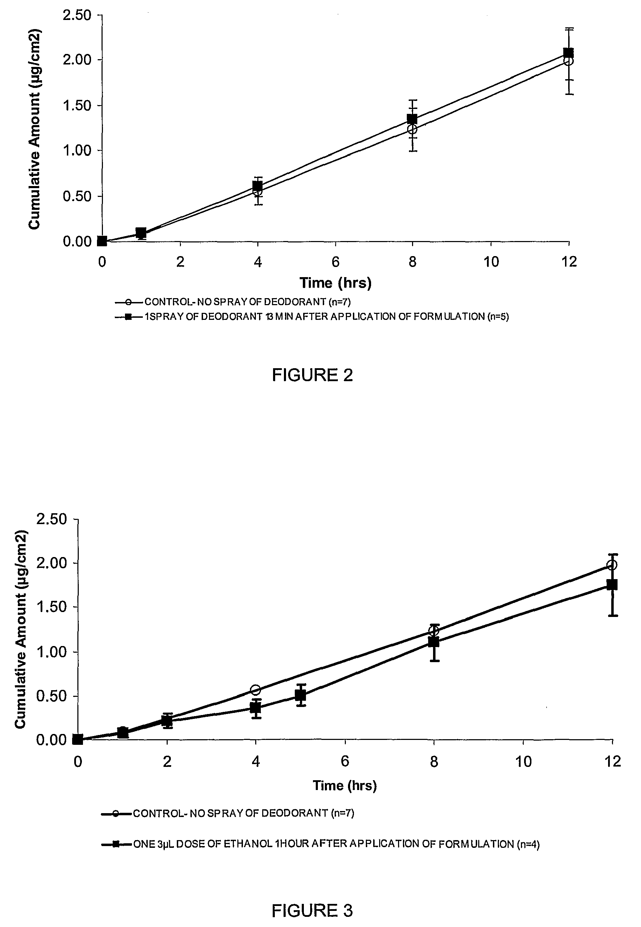

Figure 2 shows the testosterone permeation profiles obtained after application of deodorant spray to the formulation. Figure 3 shows testosterone permeation profiles obtained after application of ethanol (3 µL) to the Composition 1 formulation. In both cases, it can be seen that the control line does not differ significantly along the time period examined.

The application of either a spray of deodorant or a finite-dose of neat EtOH did not appear to have an effect on the skin permeation of testosterone from a 5µL dose of the lotion. Therefore it was concluded that the application of deodorant to the skin after application of the testosterone lotion will not impede transdermal penetration of testosterone.

### Example 3

This example investigated the cumulative testosterone and estradiol permeation through human skin *in vitro* when included in a commercial deodorant.

Finite-dose *in vitro* diffusion studies were undertaken using heat-separated human, female abdominal epidermis. These experiments were performed over 24 hours using stainless steel, flow through diffusion cells based on those described previously (Cooper, E.R. J. Pharm. Sci. 1984, 73, 1153-1156) except that the cell was modified to increase the diffusion area to 1.0cm². The formulations were applied using a finite dose technique (Franz, T.J. Curr. Probl. Dermatol., 1978, 7, 58-68) to mimic clinical dosing conditions at an applied dose volume of 5µL /cm². A piece of stainless steel wire mesh was placed directly below the skin in the receptor chamber of the of the diffusion cell to maintain a turbulent flow of receptor solution below the skin. The diffusion cells were maintained at a flow rate of approximately 1.0 mL/ cm²/hr by a microcassette peristaltic pump (Watson Marlow 505S UK). The cells were kept at 32 ± 0.5°C by a heater bar and the samples were collected into appropriately sized plastic vials on an automated fraction collector (Isco Retriever II, Lincoln, NE) at specified intervals. The receptor solution (20%v/v EtOH in 0.002%w/v NaN₃) maintained sink conditions below the skin. Two formulations were used that consisted of:
- Composition 2: 1% w/v testosterone, 5%w/v octisalate, 32% v/v EtOH to 100% v/v Deodorant (Rexona Essentials for Men 'Dry' Antiperspirant Deodorant Spray, Unilever, Australia, BN: 6030 10793)
- Composition 3: 0.5% w/v E2 estradiol, 5% w/v octisalate, 52% v/v EtOH to 100% v/v Deodorant (Rexona Activreserve 'Classic Silk' Antiperspirant Deodorant Spray, Unilever, Australia, BN: 6054 11280)
*Note: extra ethanol was required in each of the formulations in order for the octisalate to remain miscible. The amount of ethanol differed between formulations* as *the deodorants used differed.*

The amount of testosterone (TES) and estradiol (E2) that permeated the skin was quantified using a HPLC method.

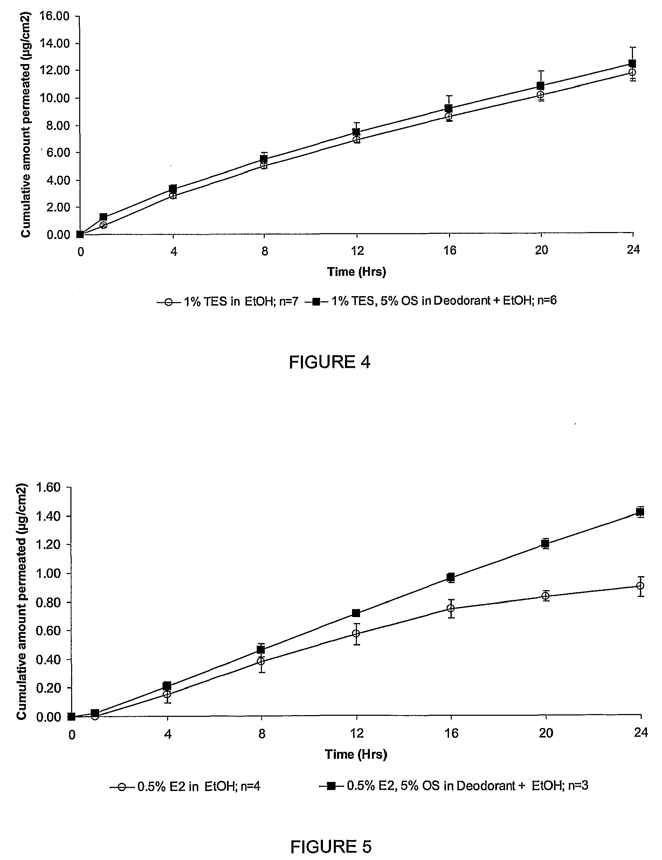

The permeation of TES through human epidermis *in vitro* was unaltered when the drug was added, with octisalate (OS) in an EtOH solution, to a commercial deodorant compared to its permeation from an ethanolic solution without the penetration enhancer. Figure 4 shows the TES permeation profiles obtained from the application of Composition 2 compared with application of a control. There is no significant difference in the curves produced. With composition 3, the permeation of E2 through human epidermis *in vitro* was significantly improved, when the drug was added, with OS in an EtOH solution, to a commercial deodorant compared to its permeation from an ethanolic solution without the penetration enhancer. Figure 5 shows the E2 permeation profiles obtained from the application of Composition 3, compared with application of a control.

The improved permeation is apparent after only 4 hours, and the difference becomes more significant with time particularly after 16 hours from dose.

The *in vitro* skin permeation of TES or E2 from an ethanolic solution comprised of OS and a commercial deodorant was therefore comparable to, if not higher than, its permeation from the ethanolic solutions comprised of the drug alone. This demonstrates that the addition of ingredients typically found in commercial deodorants to the formulations did not inhibit transdermal permeation of testosterone or estradiol.

### Example 4

A study was conducted to assess the antiperspirant and deodorant characteristics of Composition 1 without added antiperspirant or deodorants. In this study, 16 male volunteers were asked to apply Composition 1 to their axilla(s) in a similar manner as in Example 1, and also to refrain from the use of other deodorants or antiperspirants on the axilla (armpits) that received Composition 1. The subjects were, however, instructed to apply deodorant if they were distressed with symptoms of sweating and to report such application. Interestingly, none of the subjects reported a need to use deodorant or antiperspirant when Composition 1 had been applied to the axilla.

## Claims

1. Use of a transdermal drug delivery composition comprising:
(a) testosterone;
(b) at least one volatile solvent;
(c) a penetration enhancer which is one or a mixture of octyl dimethyl-para-aminobenzoate, octyl para-methoxycinnamate and octyl salicylate; and
(d) at least one viscosity modulating agent;
in manufacture of a pharmaceutical composition for the treatment of testosterone deficiency in a subject by application to at least one axilla to deliver testosterone systemically; and wherein the transdermal drug delivery composition is for application without being covered by adhesives, adhered patches, adhered bandages or films.

2. A transdermal drug delivery composition comprising:
(a) testosterone;
(b) at least one volatile solvent;
(c) a penetration enhancer which is one or a mixture of octyl dimethyl-para-aminobenzoate, octyl para-methoxycinnamate and octyl salicylate; and
(d) at least one viscosity modulating agent;
for use in the treatment of testosterone deficiency in a subject by applying the composition to at least one axilla to deliver testosterone systemically; wherein the transdermal drug delivery composition is for application without being covered by adhesives, adhered patches, adhered bandages or films.

3. The use according to claim 1 or the transdermal drug delivery composition according to claim 2, wherein the composition is for the treatment of testosterone deficiency in men.

4. The use according to claim 1 or the transdermal drug delivery composition according to claim 2, wherein the transdermal drug delivery composition is for treatment of androgen deficiency in adult males.

5. The use according to claim 1 or the transdermal delivery composition according to claim 2, wherein the penetration enhancer is present in an amount of 0.01 to 15% of the total composition. 1

6. The use according to claim 1 or the transdermal delivery composition according to claim 2, wherein the volatile solvent is a lower alkyl alcohol or mixture of such alcohols.

7. The use according to claim 1 or the transdermal delivery composition according to claim 2, wherein the volatile solvent has a vapour pressure above 35 mm Hg at atmospheric pressure and at a temperature of 32°C.

8. The use according to claim 1 or the transdermal delivery composition according to claim 2, wherein the volatile solvent is selected from the group consisting of ethanol, ethyl acetate, isopropanol, acetone, ethyl formate, methyl acetate, methyl ethyl ketone, pentane and chloroform or mixtures thereof in the range of 40 to 99% v/v of the composition.

9. The use, according to claim 1, or the transdermal delivery composition according to claim 2, wherein the volatile solvent is present at 80% w/w or more.

10. The use according to claim 1 or the transdermal drug delivery composition according to claim 2, wherein the composition comprises over 80% alcohol and the viscosity modulating agent is polyvinylpyrrolidone (PVP) in an amount of from 1% to 3%.

11. The use according to claim 1 or the transdermal drug delivery composition according to claim 2, wherein the composition is applied no more than once a day.

12. The use according to claim 1 or the transdermal drug delivery composition according to claim 2 which has a viscosity of less than 300 centipoise.

13. The use according to claim 1 or the transdermal drug delivery composition according to claim 2, wherein the composition is for achieving a testosterone blood level of at least 200 ng/dL.

## Patentansprüche

1. Verwendung einer transdermalen Arzneimittelzuführzusammensetzung, umfassend:
(a) Testosteron;
(b) mindestens ein flüchtiges Lösungsmittel;
(c) einen Penetrationsverstärker, der eines aus Octyldimethyl-para-amino-benzoat, Octyl-para-methoxycinnamat und Octylsalicylat oder ein Gemisch davon ist; und
(d) mindestens ein viskositätsmodulierendes Mittel;
zur Herstellung einer pharmazeutischen Zusammensetzung für die Behandlung von Testosteron-Mangel bei einem Subjekt durch Applikation auf mindestens eine Axilla zum systemischen Zuführen von Testosteron, und wobei die transdermale Arzneimittelzuführzusammensetzung für eine Applikation vorgesehen ist, ohne von Klebstoffen, anhaftenden Pflastern, anhaftenden Verbänden oder Filmen bedeckt zu sein.

2. Transdermale Arzneimittelzuführzusammensetzung, umfassend:
(a) Testosteron;
(b) mindestens ein flüchtiges Lösungsmittel;
(c) einen Penetrationsverstärker, der eines aus Octyldimethyl-para-aminobenzoat, Octyl-para-methoxycinnamat und Octylsalicylat oder ein Gemisch davon ist; und
(d) mindestens ein viskositätsmodulierendes Mittel;
für eine Verwendung bei der Behandlung von Testosteron-Mangel bei einem Subjekt durch Applikation der Zusammensetzung auf mindestens eine Axilla zum systemischen Zuführen von Testosteron, wobei die transdermale Arzneimittelzuführzusammensetzung für eine Applikation vorgesehen ist, ohne von Klebstoffen, anhaftenden Pflastern, anhaftenden Verbänden oder Filmen bedeckt zu sein.

3. Verwendung gemäß Anspruch 1 oder transdermale Arzneimittelzuführzusammensetzung gemäß Anspruch 2, wobei die Zusammensetzung für die Behandlung von Testosteron-Mangel bei Männern vorgesehen ist.

4. Verwendung gemäß Anspruch 1 oder transdermale Arzneimittelzuführzusammensetzung gemäß Anspruch 2, wobei die transdermale Arzneimittelzuführzusammensetzung für eine Behandlung von Androgen-Mangel bei erwachsenen Männern vorgesehen ist.

5. Verwendung gemäß Anspruch 1 oder transdermale Zuführzusammensetzung gemäß Anspruch 2, wobei der Penetrationsverstärker in einer Menge von 0,01 bis 15% der Gesamtzusammensetzung vorhanden ist.

6. Verwendung gemäß Anspruch 1 oder transdermale Zuführzusammensetzung gemäß Anspruch 2, wobei das flüchtige Lösungsmittel ein Niederalkylalkohol oder ein Gemisch solcher Alkohole ist.

7. Verwendung gemäß Anspruch 1 oder transdermale Zuführzusammensetzung gemäß Anspruch 2, wobei das flüchtige Lösungsmittel einen Dampfdruck von über 35 mm Hg bei Atmosphärendruck und bei einer Temperatur von 32°C aufweist.

8. Verwendung gemäß Anspruch 1 oder transdermale Zuführzusammensetzung gemäß Anspruch 2, wobei das flüchtige Lösungsmittel aus der Gruppe, bestehend aus Ethanol, Ethylacetat, Isopropanol, Aceton, Ethylformiat, Methylacetat, Methylethylketon, Pentan und Chloroform, oder Gemischen davon in dem Bereich von 40 bis 99% Vol./Vol. der Zusammensetzung ausgewählt ist.

9. Verwendung gemäß Anspruch 1 oder transdermale Zuführzusammensetzung gemäß Anspruch 2, wobei das flüchtige Lösungsmittel bei 80% Gew./Gew. oder mehr vorhanden ist.

10. Verwendung gemäß Anspruch 1 oder transdermale Arzneimittelzuführzusammensetzung gemäß Anspruch 2, wobei die Zusammensetzung über 80% Alkohol umfasst und das viskositätsmodulierende Mittel Polyvinylpyrrolidon (PVP) in einer Menge von 1 % bis 3% ist.

11. Verwendung gemäß Anspruch 1 oder transdermale Arzneimittelzuführzusammensetzung gemäß Anspruch 2, wobei die Zusammensetzung nicht mehr als einmal pro Tag appliziert wird.

12. Verwendung gemäß Anspruch 1 oder transdermale Arzneimittelzuführzusammensetzung gemäß Anspruch 2, die eine Viskosität von weniger als 300 Centipoise aufweist.

13. Verwendung gemäß Anspruch 1 oder transdermale Arzneimittelzuführzusammensetzung gemäß Anspruch 2, wobei die Zusammensetzung zum Erreichen eines Testosteron-Blutspiegels von mindestens 200 ng/dL vorgesehen ist.

## Revendications

1. Utilisation d'une composition pour la délivrance transdermique de médicament comprenant:
(a) de la testostérone;
(b) au moins un solvant volatil;
(c) un renforçateur de pénétration qui est un mélange de diméthyl-para-aminobenzoate d'octyle, para-méthoxycinnamate d'octyle et salicylate d'octyle, ou l'un d'entre eux; et
(d) au moins un agent modulant la viscosité;
dans la préparation d'une composition pharmaceutique pour le traitement de la déficience en testostérone chez un sujet par application à au moins une aisselle pour délivrer la testostérone par voie systémique; et dans laquelle la composition pour délivrance transdermique de médicament est pour une application sans recouvrement par adhésifs, timbres fixés par adhérence, bandages fixés par adhérence ou films.

2. Composition pour la délivrance transdermique de médicament comprenant:
(a) de la testostérone;
(b) au moins un solvant volatil;
(c) un renforçateur de pénétration qui est un mélange de diméthyl-para-aminobenzoate d'octyle, para-méthoxycinnamate d'octyle et salicylate d'octyle, ou l'un d'entre eux; et
(d) au moins un agent modulant la viscosité;
pour utilisation dans le traitement de la déficience en testostérone chez un sujet par application de la composition à au moins une aisselle pour délivrer la testostérone par voie systémique; tandis que la composition pour délivrance transdermique de médicament est pour application sans être recouverte par des adhésifs, des timbres fixés par adhérence, des bandages fixés par adhérence ou des films.

3. Utilisation selon la revendication 1 ou composition pour la délivrance transdermique de médicament selon la revendication 2, dans laquelle la composition est pour le traitement de la déficience en testostérone chez les hommes.

4. Utilisation selon la revendication 1 ou composition pour la délivrance transdermique de médicament selon la revendication 2, dans laquelle la composition pour la délivrance transdermique de médicament est pour le traitement de la déficience en androgènes chez les mâles adultes.

5. Utilisation selon la revendication 1 ou composition pour la délivrance transdermique selon la revendication 2, dans laquelle le renforçateur de pénétration est présent en une quantité de 0,01 à 15% de la composition totale.

6. Utilisation selon la revendication 1 ou composition pour la délivrance transdermique selon la revendication 2, dans laquelle le solvant volatil est un alcool d'alkyle inférieur ou un mélange de tels alcools.

7. Utilisation selon la revendication 1 ou composition pour la délivrance transdermique selon la revendication 2, dans laquelle le solvant volatil a une pression de vapeur supérieure à 35 mm de Hg à la pression atmosphérique et à une température de 32°C.

8. Utilisation selon la revendication 1 ou composition pour la délivrance transdermique selon la revendication 2, dans laquelle le solvant volatil est choisi dans le groupe consistant en éthanol, acétate d'éthyle, isopropanol, acétone, formiate d'éthyle, acétate de méthyle, cétone d'éthyle et de méthyle; pentane et chloroforme ou des mélanges de ceux-ci dans l'intervalle de 40 à 99% en volume/volume de la composition.

9. Utilisation selon la revendication 1, ou composition pour la délivrance transdermique selon la revendication 2, dans laquelle le solvant volatil est présent à 80% en poids/poids ou plus.

10. Utilisation selon la revendication 1 ou composition pour la délivrance
transdermique de médicament selon la revendication 2, dans laquelle la composition comprend plus de 80% d'alcool et l'agent modulant la viscosité est la poly(pyrrolidone de vinyle) (PVP) en une quantité allant de 1 % à 3%.

11. Utilisation selon la revendication 1 ou composition pour la délivrance
transdermique de médicament selon la revendication 2, dans laquelle la composition est appliquée pas plus d'une fois par jour.

12. Utilisation selon la revendication 1 ou composition pour la délivrance
transdermique de médicament selon la revendication 2, qui a une viscosité inférieure à 300 centipoises.

13. Utilisation selon la revendication 1 ou composition pour la délivrance
transdermique de médicament selon la revendication 2, dans laquelle la composition est pour l'obtention d'un taux sanguin de testostérone d'au moins 200 ng/dL.
